Europäisches Patentamt

European Patent Office

Office européen des brevets

(11) Publication number: **0 437 499 B1**

# EUROPEAN PATENT SPECIFICATION

(45) Date of publication of patent specification: **22.12.93**  (51) Int. Cl.⁵: **A61L  2/04**, C12H 1/18, A23L 3/02

(21) Application number: **89911538.0**

(22) Date of filing: **04.10.89**

(86) International application number: **PCT/DK89/00230**

(87) International publication number: **WO 90/03806 (19.04.90 90/09)**

(54) **METHOD AND APPARATUS FOR THE PASTEURIZATION OF A CONTINUOUS LINE OF PRODUCTS.**

(30) Priority: **05.10.88 DK 5578/88**

(43) Date of publication of application: **24.07.91 Bulletin  91/30**

(45) Publication of the grant of the patent: **22.12.93 Bulletin  93/51**

(84) Designated Contracting States: **AT BE CH DE FR GB IT LI LU NL SE**

(56) References cited:
**GB-A- 2 182 542**
**US-A- 4 490 401**

(73) Proprietor: **SANDER HANSEN A/S Fabriksparken 24 DK-2600 Glostrup(DK)**

(72) Inventor: **FRYDKJAER, Karsten Fuglebakkevej 92, 6.3. DK-2000 Kobenhavn F(DK)**

(74) Representative: **Birkeholm, Mogens et al Larsen & Birkeholm ApS Skagensgade 64 P.O. Box 200 DK-2630 Taastrup (DK)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3. 10/3.6/3.3. 1)

EP 0 437 499 B1

## Description

The invention relates to a method for the pasteurization of a continuous line of products in an apparatus which has a heating area, a pasteurization area and a cooling area together with means of transport for carrying the products in an even motion through the areas in the said order from the inlet of the apparatus to the outlet, whereby the heating, the pasteurization and the cooling is effected through beat transfer between the products and a fluid, preferably water, which is sprinkled over the products, which areas are divided in zones extending in the direction of the motion of the products, and in which the temperature of the fluid in the particular zone is adapted to the desired sequence of the heat transfer for the zone.

The invention also concerns an apparatus for use in the implementation of the said method, which apparatus has a heating area, a pasteurization area and a cooling area together with means of transport for carrying the products in an even motion through the areas in the said order from the inlet of the apparatus to the outlet, whereby the heating, the pasteurization and the cooling is effected through heat transfer between the products and a fluid, preferably water, which is sprinkled over the products, which areas are divided into zones extending in the direction of the motion of the products, and in which the temperature of the fluid in the particular zone is adapted to the desired sequence of the heat transfer for the zone.

In the production of products, which can be spoiled by bacterial flora, it is well known to destroy the bacterial flora by pasteurization, which in the case of a continuous line of products, takes place in a so called tunnel pasteurization apparatus.

A continuous line of products, which necessarily has to be pasteurized to achieve keeping qualities, is well known from the brewery industries in the shape of beer which is packaged in bottles.

The known apparatus for tunnel pasteurization are of the kind in which the products are being pasteurized in an uniformly progressing motion from an inlet to an outlet. During the motion the products will be heated in such a way that pasteurization takes place and normally a cooling will also take place in the end to stop the process of pasteurization. Therefore such apparatuses can be divided in a heating area, a pasteurization area and a cooling area in the said order. It is not necessarily the case, that the known apparatus comprise a heating and a cooling area, but in the brewery industries this will normally be the case, since the purpose of a gradual heating and a gradual cooling partly is to avoid destruction of the containers for the products, normally glass bottles, through abrupt changes of temperature, and partly is to exploit a heat exchange between the heating areas and the cooling areas.

The heat transfer to the products in the containers is normally achieved by sprinkling a fluid, preferably water, over the containers while they are moved through the apparatus, typically on a conveyor belt through which the sprinkling fluid can pass. In such cases a vessel is placed under the conveyor belt from which the sprinkling fluid can be pumped to a higher level so that the sprinkling can continue.

At one or more places in the apparatus means are provided for heat exchange between cooler and hotter sprinkling fluid to save energy. This heat exchange can for instance be executed through heating coils or tubes in the vessels or by placing heat exchangers in the pumping tubes. Cold or hot fluid may possibly be added directly to the sprinkling fluid.

To be able to differentiate the temperatures of the sprinkling fluids the said three areas of the apparatus are furthermore divided in zones. Typically the heating area may consist of three zones, the pasteurization area of two and the cooling area of three or four zones, so that the containers are first exposed to a stepwise increasing temperature of the sprinkling fluid which results in an evenly increasing temperature of the products in the pasteurization area, whereafter the products at last in the cooling area are exposed to a stepwise decreasing temperature of the sprinkling fluid which results in a evenly decreasing temperature of the products.

If the pasteurization area in the known apparatus as mentioned above is divided in two zones, the first zone is designated as the overheating zone while the following is designated as the pasteurization zone. The sprinkling fluid in the overheating zone is warmer than the sprinkling fluid in the pasteurization zone as the aim is that the products shall substantially obtain the temperature of the pasteurization zone when they pass into this zone.

To achieve the proper pasteurization the pasteurization zone is normally three to five times longer than the overheating zone.

The various sprinkling temperatures are carefully adapted to the product, to the length of the zones of the apparatus and to the speed of the conveyor belt to ensure that the product will receive the prescribed degree of pasteurization.

It will be understood from this that the known type of apparatus is based on a constant speed of the conveyor belt.

The described known apparatus is simple in its mechanical construction and therefore extremely reliable. Nevertheless a possible function stoppage of succeeding considerably more complicated apparatus could cause the conveyor belt of the pasteurization apparatus to be stopped or at least slowed down to avoid an accumulation of products.

The result of this is that products placed in the pasteurization zone will be over-pasteurizated unless the temperatures of the sprinkling fluids are lowered.

If these temperatures are lowered products placed in the apparatus, when the function stoppage occurs, will be under-pasteurizated which is worse than over-pasteurization, as under-pasteurization causes a substantial reduction of the keeping qualities.

A reduction of the temperature of the sprinkling fluid will result in a very great loss of energy in connection with the draining off of hot sprinkling fluid and introduction of cold fluid and when the apparatus later is returned to its stationary state of function the cold sprinkling fluid again has to be heated resulting in a further loss of energy.

It is therefore the object of this invention to indicate a method of controlling the temperature of the sprinkling fluid and an apparatus for use in connection with the method by which the above mentioned disadvantages of respectively over- and under-pasteurization of the products can be avoided at a function stoppage during the treatment of the products and by which also a lower expenditure of energy is obtained in comparison with the known techniques.

This purpose is obtained by a method of the above mentioned description which method is characteristic according to the invention in that a previously fixed uniform temperature of the sprinkling fluid is established in the pasteurization area and that means are provided which during a slowing down or a function stoppage of the conveyor belt can change the sequence of the heat transfer in the zones of the pasteurization area by cross guiding the down flowing sprinkling fluid between the zones, whereby the colder products in the pasteurization area are used to cool the warmer products through a heat equalization in the area by means of the sprinkling fluid so that an extra introduction of cold sprinkling fluid or a draining off of warm sprinkling fluid can be avoided.

The idea of the invention is to employ the relatively cold products in the pasteurization area for cooling the relatively warmer products through a kind of temperature equalizing in the area through the sprinkling fluid in such a way that at the stationary state of function of the apparatus, i. e. when the conveyor belt is moved at the prescribed speed, a different sequence of the heat transfer between the sprinkling fluid and the products is obtained, which sequence in the following will be denoted as the temperature profile.

It is the temperature profile which determines the degree of pasteurization, but as the various temperature profiles can result in the same degree of pasteurization there is a free choice in this field.

With the previously known method a substantially constant temperature profile is obtained in the whole of the pasteurization area except for a short stretch at the beginning of the pasteurisation zone. Hereby all the products in the area obtain approximately the same temperature.

On the other hand with the method according to the invention an evenly increasing temperature profile is obtained in the whole pasteurization area, so that a greater amount of cold products are available for cooling a smaller amount of warm products.

With the method according to the invention some valves in pipes between the vessels placed under the conveyor belt in the various zones of the pasteurization area are opened when a stoppage occurs, so that sprinkling fluid can be transported between the said zones in the pasteurization area.

When cooling down is no longer needed the means may be readjusted to their normal position and in this position conduct the sprinkling fluid through the apparatus as in the stationary state of function or some of the means can be adjusted and heat supplied to the sprinkling fluid. This will depend on the duration of the stoppage.

The invention also relates to an apparatus for use in the implementation of the said method, which apparatus is of the kind mentioned in the introduction and this apparatus is characteristic according to the invention in that the apparatus has means for establishing a previously fixed uniform temperature of the sprinkling fluid in the pasteurization area and means which are constructed for exchange down flowed sprinkling fluid between the zones in the pasteurization area by cross guiding down flowed sprinkling fluid between the zones.

The subclaims indicate respectively preferred ways of proceeding and preferred apparatus for use in implementing the method and the expedience of the means indicated in the sub-claims will be apparent from that which is explained below.

The method according to the invention will be explained in more detail in the following, partly with reference to an embodiment of the apparatus according to the invention, and partly with reference to the drawings, in which

fig. 1    shows curves of temperatures in a sprinkling fluid in products which are being pasteurized and the degree of pasteurization in the said products in a known apparatus in the stationary state of function,

fig. 2    shows corresponding curves in an apparatus for implementing the method according to the invention in the stationary state of function of this apparatus,

fig. 3    shows corresponding curves in the apparatus indicated in fig. 2 during a function stoppage.

In the following the invention is explained on the basis of tunnel pasteurization apparatuses which are being used in the brewery industries for pasteurising Pilsener beer to enhance the keeping qualities which apparatuses enter into a tapping column which for instance produces Pilsener beer in glas bottles.

Depending on the size of the tunnel pasteurization apparatus and the size of the bottles of Pilsener beer between 2.500 and 120.000 bottles can be pasteurized per hour with typical passage periods of 50-55 minutes.

It is prescribed for Pilsener beer that it has to have a degree of pasteurization which is measured in Pasteurization Units. Such units are designated in an abbreviated form as PU.

The number of units of pasteurisation can be calculated from the equation:

$$PU = 10^{\frac{TP-X}{Z}} \cdot t,$$

in which X and Z are production specific constants.

For beer the constant $X = 60\,°C$ and $Z = 6{,}95$; hereby the equation for beer becomes:

$$PU = 1{,}393^{TP-60°C} \cdot t,$$

in which TP is the temperature of the products of pasteurization and t is the time measured in minutes. If TP for example is a constant $60\,°C$ the product will pick up 1 unit of pasteurization per minute.

For a correct pasteurization of Pilsener beer 15 PU are prescribed, and it will be understood from the equation above that the prescibed amount of PU can be obtained at various combinations of pasteurization temperatures and pasteurization times which combinations or sequences can be delineated graphically by the so called temperature profile as the changes of temperature are implemented by means of a sprinkling fluid with a suitable temperature.

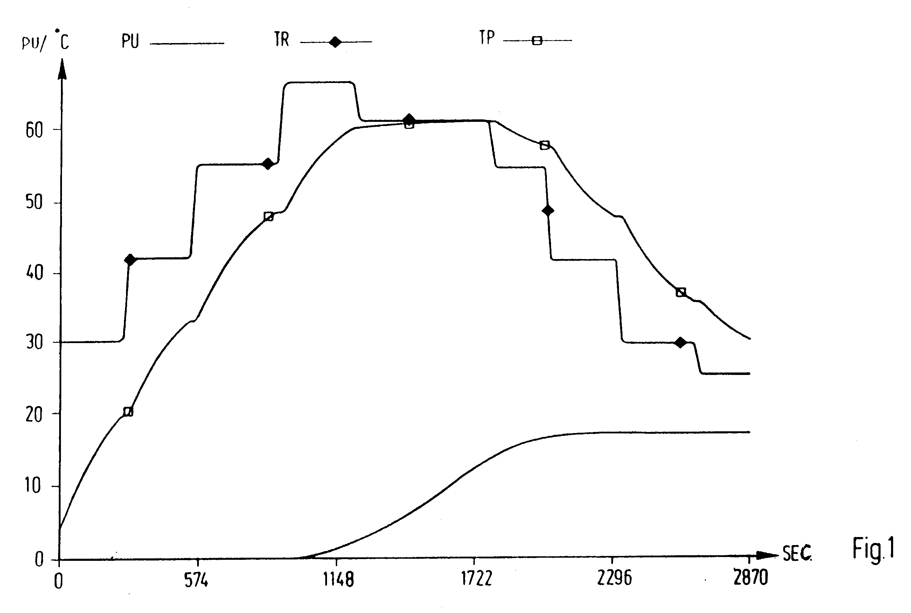

In fig. 1 is graphically shown the temperature profile for the pasteurization of Pilsener beer in a known apparatus. Time is marked in seconds along the abscissa and temperature is marked in °C along the ordinate.

Three curves are delineated in the figure:
A first curve designated TR for indicating the temperature of the sprinkling fluid, a second curve designated TP for indicating the temperature of the Pilsener beer and a third curve disignated PU for indicating the number of units of pasteurization supplied to the Pilsener beer. It should be mentioned that in the figure the graduations of the PU are corresponding to the graduations of the temperatures.

The Pilsener beer is contained in bottles which standing on a conveyor belt are transported at a uniform speed at the time $t = 0$ into the heating area of the apparatus, which area is divided in three zones in which the sprinkling fluid has different temperatures. When the sprinkling fluid in a zone has flowed down over the bottles of Pilsener beer it penetrates the openings in the conveyor belt and is collected in vessels from which it is circulated for renewed down flowing. In the first zone the $TR = 30\,°C$, in the second zone the $TR = 42\,°C$ and in the third zone the $TR = 55\,°C$ and it is possible to read that a bottle of Pilsener Beer is c. 15 minutes to pass the three zones of the heating area. During this passage the temperature of the Pilsener beer rises in an evenly ascending curve from $TP = 4\,°C$ to $TP = 48\,°C$.

The purpose of dividing the heating area in the said three zones with different temperatures of the sprinkling fluid is among other things to protect the glass containers against sudden changes of temperature which could break the glass containers.

Although a division into three zones is described here, it will be understood that fewer zones such as two heating zones or more heating zones for example four or five are only a possible choice for the person skilled in the art, depending on the kind of container, the nature of the product and the necessary temperature of pasteurization.

The containers with the Pilsener beer will be transported further through the apparatus at the above mentioned regular speed and into the pasteurization zone which here is divided into an overheating zone and a pasteurization zone.

In the overheating zone the sprinkling fluid has a temperature $TR = 67\,°C$ whereby the temperature of the Pilsener beer rises from the said $48\,°C$ at the passage between the heating zone and the overheating zone to c. $60\,°C$ at the passage between the last mentioned zone and the pasteurization zone. In the pasteurization zone the sprinkling fluid has a temperature $TR = 62\,°C$ so that the temperature TP of the Pilsener beer by and large is not changed during the passage of the pasteurization zone, which is shown in the curve by a break at the said passage.

It will be noticed that the amount of PU has increased from c. 0 at the passage between the heating zone and the overheating zone to 13-14 PU when the containers leave the pasteurization zone.

The containers with Pilsener beer pass further on through the apparatus and into the cooling zone where the Pilsener beer is to be cooled. If the Pilsener beer is not cooled the amount of PU will continue to increase, cf. the above mentioned equation for PU.

In fig. 1 the cooling area is divided into four zones in which the temperatures of the sprinkling fluid are respectively $TR = 54\,°C$, $TR = 42\,°C$, $TR = 29\,°C$ and $TR = 25\,°C$ so that the Pilsener beer in the first cooling zone is still being pasteurized as a result of the temperature of the products. The degree of pasteurization of the Pilsener beer thereby increases to the prescribed 15 PU and the Pilsener beer at the outlet of the pasteurization apparatus has a temperature which from $TP = 62\,°C$ has decreased evenly to $TP = 30\,°C$ so that the process of pasteurization has practically stopped and has been accomplished in c. 50 minutes.

In the known pasteurisation apparatus a heat exchange is performed for energy purposes between the sprinkling fluid in the heating area and in the cooling area. The heat exchange can also be performed between the zones in the above mentioned areas.

If it is necessary to stop the conveyor belt because a function stoppage has occurred further along in the tapping column, Pilsener beer, which is placed in the hotter zones of the heating area, will obtain a higher degree of pasteurization, than previously stipulated for the stationary state of function of the known apparatus, already before it arrives at the pasteurization area. By a later restart of the apparatus this Pilsener beer therefore becomes over-pasteurized, i. e. PU > 17.

Pilsener beer which is placed in the pasteurization area at the time of a function stoppage will be exposed to the hot sprinkling fluid during the whole duration of the function stoppage and will therefore obtain a degree of pasteurization which is far beyond the prescribed.

So far the problems of a function stoppage have been solved by supplying cold sprinkling fluid so that the temperature of the Pilsener beer is decreased whereby the growth in the degree of pasteurization is checked.

However this kind of solution to the problem causes a large consumption of cooling water and a risk of under-pasteurization of the Pilsener beer until the apparatus after restart has again reached its stationary state of function. Besides this solution of the problem is connected with a large loss of energy and of sprinkling fluid.

It should be remembered that at the commencement of a function stoppage one is unaware of the duration of the stoppage and it is therefore difficult to introduce the right measures at the pasteurization apparatus to mend the disadvantages caused by the stoppage.

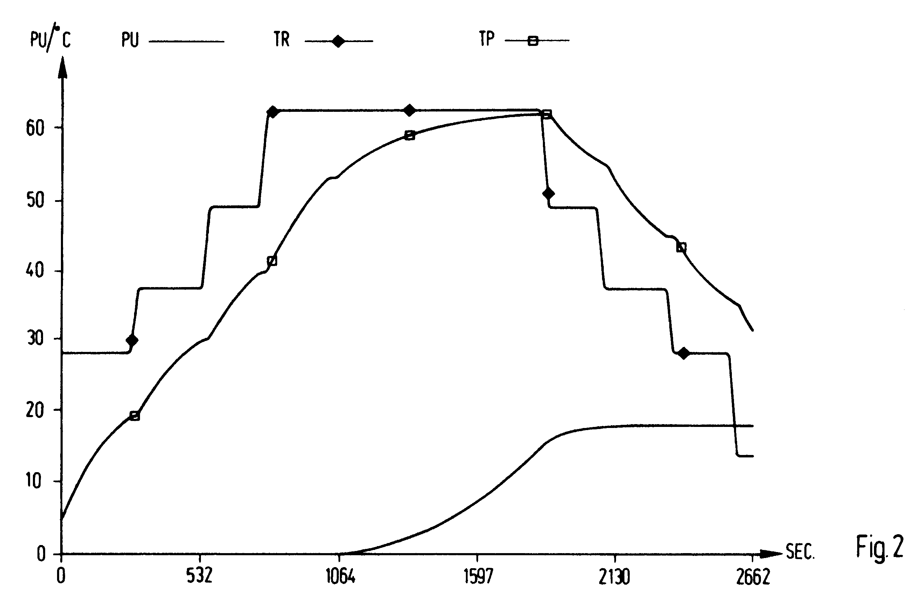

In the method according to the invention which is explained in the following with reference to fig. 2 the apparatus is also divided in a heating area with three zones, a cooling area with four zones and a pasteurization area.

The pasteurization area is divided in a first and a second zone, but the sprinkling fluid in these two zones has the same temperature even if the sprinkling fluids in the two zones are kept apart during the stationary state of function of the apparatus.

It could well be imagined that the pasteurisation area consisted of only one zone. The division in two zones exists exclusively because thereby a possibility is obtained to make a differentiated heating, which by the way is not always necessary.

Fig. 2 shows the same three curves as fig. 1, but it will be understood that the temperatures TR of the sprinkling fluid in the three areas has other values whereby the temperature of the Pilsener beer during the stationary state of the apparatus increases evenly from $TP = 5\,°C$ to $TP = 62\,°C$ at the passage between the pasteurization area and the cooling area, from which TP after the passage of a break decreases evenly during the transport towards the exit of the apparatus where $TP = 30\,°C$.

By comparing the pasteurization areas in fig. 1 and 2 it will be understood that in the method shown in fig. 2 there will be considerably more cooler than warmer containers with Pilsener beer than is the case in fig. 1, while nevertheless this change of the temperature profile achieves the same degree of pasteurisation, i. e. PU = 15.

When a function stoppage occurs this amount of cooler containers with Pilsener beer is utilized for cooling the warmer containers as means are activated which can conduct sprinkling fluid from the vessel under the first zone to the down flow in the second zone.

The sprinkling fluid from the vessel under the first zone has been cooled by passage over the cooler containers wherefore this sprinkling fluid can be utilized for cooling the warmer containers. Correspondingly means are activated which can conduct sprinkling fluid from the vessel under the second zone to flow down over containers in the first zone so that this sprinkling fluid is also cooled.

Furthermore the supply of heat which usually is conducted to the sprinkling fluid in the pasteurization area may according to the circumstances be disconnected. Although at a function stoppage of a longer duration it may again be necessary to supply heat.

The said means for conducting sprinkling fluid from one zone to another can consist of pipelines known in the art and provided with pumps and valves which can be controlled in a known way.

To control the temperatures of the sprinkling fluid it is necessary to employ process computers which in a short time can perform the necessary operations.

The computer must be able to adjust the valves and pumps which regulates the sprinkling temperatures in accordance with the speed of the conveyor belt, a first knowledge about how fast it is possible to change the temperature TP of the sprinkling fluid and a second knowledge about which degree of pasteurization each bottle of Pilsener beer has received at the actual point in time.

The speed of the conveyor belt can be fed continuously to the computer by known means.

The above mentioned first knowledge must be found through computation which is later controlled and adjusted empirically, whereafter it can be entered into the computer as a kind of database.

The said second knowledge which is a knowledge about the actual temperature profile in the apparatus has to be provided through a continuous integration of the differential equations:

$$\text{I.} \qquad \frac{dPU}{dt} = 1,393^{TP-60^{\circ}C}$$

and

$$\text{II.} \qquad \frac{dTP}{dt} = KP \ (TR - TP)$$

where KP is a heat transfer coefficient and where, as mentioned above, PU is the amount of pasteurization units, TP is the temperature of the Pilsener beer and TR is the temperature of the sprinkling fluid.

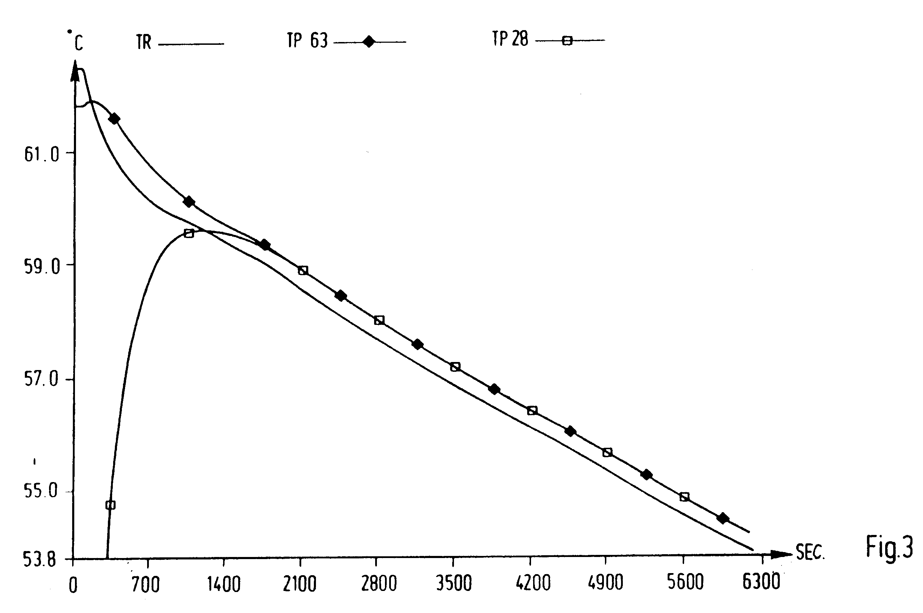

Fig. 3 shows curves of the sequence of temperature in the first and second zone of the pasteurization area during a function stoppage in an apparatus for use in the implementation of the method according to the invention. The curve, which is designated TR, indicates the sequence of temperature of the sprinkling fluid in both zones when this is cross pumped between these zones and the usual supply of heat is discontinued, the curve TP63 indicates the temperature sequence in the product in a container which is placed at the passage between the second pasteurization zone and the cooling area, and the curve TP28 indicates the sequence of temperature in a product in a container which at the time of the commencement of the function stoppage is placed at the passage between the heating area and the first pasteurization zone.

The numbers 28 and 63 therefore indicate the positions of two specific containers in the apparatus at the commencement of the function stoppage, and it will be understood that all the other containers in the apparatus are connected to corresponding position indications in such a way that the said computer can achieve a general control of all the containers in the apparatus.

The curve TR falls rapidly in the beginning as the sprinkling fluid is cooled strongly by the flow down over the large number of cooler containers in the first zone and especially in the first part of this. Thereafter the curve TR declines evenly in correspondance with the heat transfer to the surroundings.

The curve TP63 shows in the beginning a slight increase which is caused by the heat transfer from the containers to the Pilsener beer, whereafter the curve declines evenly as a result of the sprinkling with the increasingly colder sprinkling fluid.

The curve TP28 increases quickly in the beginning as the containers at the entrance of the first zone are sprinkled with fluid which is considerably warmer than the containers and the Pilsener beer contained in them. Thereafter the curve TP28 flattens out so that it later gradually becomes merged with the curve TP63, whereafter the curves TP28 and TP63 will constantly show a temperature which, although it is evenly decreasing, will still be slightly higher than the temperature of the sprinkling fluid.

To optimize the method thermographs will be applied during the stationary state of function of the apparatus, which thermographs after passage through the apparatus will show the current temperature profile at any time. At a function stoppage the temperature profile in the various thermographs may be used to enhance the knowledge which the computer must receive concerning the speed of change of the temperature of the sprinkling fluid and the degree of pasteurization of the Pilsener beer.

It will be understood from the explanation above that through the method according to the invention and the herefore developed apparatus the disadvantages explained in the introduction, which are connected with a function stoppage in the known pasteurization apparatus, have been overcome.

**Claims**

1. Method for the pasteurization of a continuous line of products in an apparatus which has a heating area, a pasteurization area and a cooling area together with means of transport for carrying the products in an even motion through the areas in the said order from the inlet of the apparatus to the outlet, whereby the heating, the pasteurization and the cooling is effected through heat transfer between the products and a fluid, preferably water, which is sprinkled over the products, which areas are divided in zones extending in the direction of the motion of the products, and in which the temperature of the fluid in the particular zone is adapted to the desired sequence of the heat transfer for the zone, **characterised** in that a previously fixed uniform temperature of the sprinkling fluid is established in the pasteurization area and that means are provided which during a slowing down or a function stoppage of the conveyor belt can change the sequence of the heat transfer in the zones of the pasteurization area by cross guiding the down flowing sprinkling fluid between the zones, whereby the colder products in the pasteurization area are used to cool the warmer products through a heat equalization in the area by means of the sprinkling fluid so that an extra introduction of cold sprinkling fluid or a draining off of warm sprinkling fluid can be avoided.

2. Method according to claim 1, **characterised** in that the cross guiding is controlled by valves which are regulated by a computer.

3. Method according to claims 1 and 2, **characterised** in that the further sequence of the heat transfer is controlled by supplying heated sprinkling fluid and/or by adding heat to the sprinkling fluid.

4. Method according to claims 1-3, **characterised** in that the computer is made to control the valves on the basis of the speed of the conveyor means, the speed of change of the heat transfer in the zones of the pasteurization area and the heat transfer to the separate products.

5. Apparatus for the pasteurization of a continuous line of products which apparatus is intended for use in implementing the method according to claim 1 and has a heating area, a pasteurization area and a cooling area together with means of transport for carrying the products in an even motion through the areas in the said order from the inlet of the apparatus to the outlet, whereby the heating, the pasteurization and the cooling is effected through heat transfer between the products and a fluid, preferably water, which is sprinkled over the products, which areas are divided into zones extending in the direction of the motion of the products, and in which the temperature of the fluid in the particular zone is adapted to the desired sequence of heat transfer for the zone, **characterised** in that the apparatus has means for establishing a previously fixed uniform temperature of the sprinkling fluid in the pasteurization area and means which are constructed to exchange down flowed sprinkling fluid between the zones in the pasteurization area by cross guiding down flowed sprinkling fluid between the zones.

6. Apparatus according to claim 5, **characterised** in that the means of exchanging or cross guiding sprinkling fluid consist of pumps, pipelines between the zones and valves placed in the pipelines, and a computer which is made to control the pumps and valves.

7. Apparatus according to claims 5 and 6, **characterised** in that the means also consist of a device for supplying heated sprinkling fluid and/or a device for supplying heat to the sprinkling fluid.

8. Apparatus according to claims 5-7. **characterised** in that the computer is made to continuously registration of the speed of the conveyor belt, the speed of change of the heat transfer in the zones of the pasteurization area and the heat supplied to the individual product.

**Patentansprüche**

1. Verfahren für die Pasteurisierung einer kontinuierlichen Erzeugniskette in einer Vorrichtung mit einem Aufheizgebiet, einem Pasteurisierungsgebiet und einem Kühlgebiet, und mit Transportmitteln, weiche die Produkte mit gleichmässiger Bewegung durch die Gebiete in der oben genannten Reihenfolge vom Einlass zum Auslass der Vorrichtung führen, wobei die Produkte aufgeheizt, pasteurisiert und gekühlt werden durch Wärmeübertragung eines Fluids, vorzugsweise Wasser, mit welchem die Produkte besprüht werden, wobei die Gebiete in Richtung der Fortbewegung der Produkte in Zonen unterteilt sind, in denen die Temperatur des Fluids an die gewünschte Folge der Wärmeübertragung in der Zone angepasst ist, dadurch gekennzeichnet, dass eine vorbestimmte gleichmässige Temperatur der Sprühflüssigkeit im Pasteurisierungsgebiet eingehalten wird, und dass Mittel vorgesehen sind, welche bei einer Verringerung der Geschwindigkeit oder eines funktionsbedingten Anhaltens des Förderbandes die Reihenfolge der Wärmeübertragung in den Zonen des Pasteurisierungsgebietes dadurch ändern können, dass sie die abfliessende Sprühflüssigkeit der Zonen kreuzweise umleiten, derart dass die kühleren Produkte im Pasteurisierungsgebiet dazu verwendet werden um damit die wärmeren Produkte zu kühlen, durch den Wärmeausgleich der Sprühflüssigkeit in diesem Gebiet, wodurch eine zusätzlich Zufuhr von kalter Sprühflüssigkeit oder ein Ablassen von warmer Sprühflüssigkeit vermieden werden kann.

2. Verfahren nach Anspruch 1, dadurch gekennzeichnet, dass die kreuzweise Umleitung mittels Ventilen geregelt wird, die von einem Computer gesteuert werden.

3. Verfahren nach den Ansprüchen 1 und 2, dadurch gekennzeichnet, dass die weitere Folge der Wärmeübertragung durch eine Zufuhr von heisser Sprühflüssigkeit und/oder durch Wärmezufuhr in die Sprühflüssigkeit geregelt wird.

4. Verfahren nach den Ansprüchen 1 bis 3, dadurch gekennzeichnet, dass der Computer die Ventile nach Massgabe der Geschwindigkeit des Förderbandes, die Geschwindigkeit der Wärmeübertragung in den Zonen des Pasteurisierungsgebietes und die Wärmeübertragung auf die einzelnen Produkte regelt.

5. Vorrichtung für die Pasteurisierung einer kontinuierlichen Erzeugniskette, mit der das Verfahren nach Anspruch 1 durchgeführt werden kann, wozu die Vorrichtung ein Aufheizgebiet, ein Pasteurisierungsgebiet und ein Kühlgebiet zusammen mit Transportmitteln, welche die Produkte mit gleichmässiger Bewegung durch die Gebiete in der oben genannten Reihenfolge vom Einlass zum Auslass der Vorrichtung führen, wobei die Produkte aufgeheizt, pasteurisiert und gekühlt werden durch Wärmeübertragung eines Fluids, vorzugsweise Wasser, mit welchem die Produkte besprüht werden, wobei die Gebiete in Richtung der Fortbewegung der Produkte in Zonen unterteilt sind, in denen die Temperatur des Fluids an die gewünschte Folge der Wärmeübertragung in der Zone angepasst ist, dadurch gekennzeichnet, dass die Vorrichtung mit Mitteln versehen ist, welche ein Aufrechterhalten einer vorbestimmten gleichmässigen Temperatur der Sprühflüssigkeit im Pasteurisierungsgebiet erlauben, und dass Mittel vorgesehen sind zum kreuzweisen Austauschen der aus den einzelnen Zonen des Pasteurisierungsgebietes abfliessenden Sprühflüssigkeit zwischen den Zonen.

6. Vorrichtung nach Anspruch 5, dadurch gekennzeichnet, dass die Mittel zum kreuzweisen Austausch der Sprühflüssigkeit, Pumpen, Rohrleitungen zwischen den Zonen, und in den Rohrleitungen angeordnete Ventile, sowie einen Computer umfassen, welcher die Pumpen und Ventile steuert.

7. Vorrichtung nach den Ansprüchen 5 und 6, dadurch gekennzeichnet, dass die Mittel auch ein Gerät für die Zufuhr von erhitzter Sprühflüssigkeit und/oder ein Gerät für die Zufuhr von Wärme an die Sprühflüssigkeit umfassen.

8. Vorrichtung nach den Ansprüchen 5 bis 7, dadurch gekennzeichnet, dass der Computer so eingerichtet ist, dass er die Geschwindigkeit des Förderbandes, die Geschwindigkeit der Wärmeübertragung in den Zonen des Pasteurisierungsgebietes und die Wärmeübertragung auf die einzelnen Produkte ständig registriert.

**Revendications**

1. Procédé pour la pasteurisation d'une chaîne de produits en continu dans un appareil qui a une région de chauffage, une région de pasteurisation et une région de refroidissement ainsi que des moyens de transport des produits selon un mouvement plan à travers les régions dans l'ordre susmentionné de l'entrée de l'appareil à la sortie, le chauffage, la pasteurisation et le refroidissement étant ainsi effectués par transfert de chaleur entre les produits et un liquide, de préférence l'eau, qui est répandu en pluie fine sur les produits, ces régions étant divisées en zones s'étendant dans la direction du mouvement des produits, la température du liquide dans chaque zone étant adaptée à la séquence désirée du transfert de chaleur pour la zone, caractérisé en ce qu'une température uniforme préalablement fixée du liquide à répandre est établie dans la région de pasteurisation et en ce que l'on dispose des moyens qui, pendant un ralentissement ou un arrêt de fonctionnement de la bande transporteuse, peuvent modifier la séquence du transfert de chaleur dans les zones de la région de pasteurisation en faisant se croiser le liquide à répandre écoulé entre les zones, les produits plus froids dans la région de pasteurisation étant utilisés pour refroidir les produits plus chauds par une égalisation de chaleur dans la région au moyen du liquide à répandre de sorte qu'une introduction supplémentaire de liquide à répandre froid ou qu'une évacuation de liquide à répandre chaud puissent être évitées.

**2.** Procédé selon la revendication 1, caractérisé en ce que le mouvement croisé est effectué au moyen de vannes qui sont commandées par un ordinateur.

**3.** Procédé selon les revendications 1 et 2, caractérisé en ce que la séquence suivante de transfert de chaleur est commandée par apport de liquide à répandre chauffé et/ou par addition de chaleur au liquide à répandre.

**4.** Procédé selon les revendications 1 à 3, caractérisé en ce que l'ordinateur commande les vannes sur la base de la vitesse des moyens de transport, de la vitesse du changement de transfert de chaleur dans les zones de la région de pasteurisation et du transfert de chaleur aux produis séparés.

**5.** Appareil pour la pasteurisation d'une chaîne de produits en continu, cet appareil étant destiné à la mise en oeuvre du procédé selon la revendication 1 et ayant une région de chauffage, une région de pasteurisation et une région de refroidissement ainsi que des moyens de transport des produits selon un mouvement plan à travers les régions dans l'ordre susmentionné de l'entrée de l'appareil à la sortie, le chauffage, la pasteurisation et le refoidissement étant ainsi effectués par transfert de chaleur entre les produits et un liquide, de préférence l'eau, qui est répandu en pluie fine sur les produits, ces régions étant divisées en zones s'étendant dans la direction du mouvement des produits, la température du liquide dans chaque zone étant adaptée à la séquence désirée de transfert de chaleur pour la zone, caractérisé en ce que l'appareil a des moyens pour établir une température uniforme préalablement fixée du liquide à répandre dans la région de pasteurisation, et des moyens qui sont conçus pour échanger le liquide à répandre écoulé entre les zones dans la région de pasteurisation en faisant se croiser le liquide à répandre écoulé entre les zones.

**6.** Appareil selon la revendication 5, caractérisé en ce que les moyens pour échanger ou faire se croiser du liquide à répandre consistent en pompes, canalisations entre les zones et vannes placées dans les canalisations, et un ordinateur qui est destiné à commander les pompes et les vannes.

**7.** Appareil selon les revendications 5 et 6, caractérisé en ce que les moyens consistent également en un dispositif pour apporter du liquide à répandre chauffé et/ou un dispositif pour apporter de la chaleur au liquide à répandre.

**8.** Appareil selon les revendications 5 à 7, caractérisé en ce que l'ordinateur est conçu pour un enregistrement en continu de la vitesse de la bande transporteuse, de la vitesse de changement du transfert de chaleur dans les zones de la région de pasteurisation et de la chaleur apportée à chaque produit.

Fig.1

Fig. 2

EP 0 437 499 B1

Fig.3

EP 0 437 499 B1